# EUROPÄISCHE PATENTANMELDUNG

(11) **EP 2 735 389 A1**
(43) Veröffentlichungstag der Anmeldung: **28.05.2014**
(21) Anmeldenummer: 12194129.8
(22) Anmeldetag: 23.11.2012
(51) Int. Cl.: B22F 1/00, B22F 9/04

(54) **Verfahren zur Herstellung reiner, insbesondere kohlenstofffreier Nanopartikel**

(71) Anmelder: Universität Duisburg-Essen, 45141 Essen (DE)
(72) Erfinder: Lau, Marcus, 45130 Essen (DE); Barcikowski, Prof. Dr., Stephan, 45359 Essen (DE)
(74) Vertreter: Taruttis, Stefan Georg

(57) **Zusammenfassung**

Die Erfindung stellt ein Verfahren zur Herstellung von Nanopartikeln bereit, sowie eine Vorrichtung, die zur Herstellung der Nanopartikel eingerichtet ist. Das Verfahren zeichnet sich insbesondere dadurch aus, dass die Nanopartikel rein, insbesondere frei von organischen Kohlenstoffverbindungen, bevorzugt kohlenstofffrei, und kontinuierlich erhalten werden. Die Nanopartikel, die mit dem erfindungsgemäßen Verfahren erhältlich sind, zeichnen sich dadurch aus, dass sie ohne einen organischen Liganden in Suspension vorliegen und insbesondere bevorzugt als Suspension gegen Agglomeration stabil sind, wobei das Medium mit den darin suspendierten Partikeln frei von organischen Kohlenstoffverbindungen, insbesondere kohlenstofffrei ist.

## Beschreibung

Die vorliegende Erfindung betrifft ein Verfahren zur Herstellung von kleinen Nanopartikeln und Atomclustern, bevorzugt aus Gold, die insbesondere einen Durchmesser von kleiner als 5 nm, bevorzugt kleiner 3 nm aufweisen und bevorzugt frei von organischen Kohlenstoffverbindungen sind, insbesondere kohlenwasserstofffrei sind, z.B. als einzige Kohlenstoffverbindung CO₂, insbesondere in Wasser gelöst, z.B. als Carbonatanionen, aufweisen und besonders bevorzugt kohlenstofffrei sind, z.B. in wässriger Zusammensetzung. Weiter betrifft die Erfindung die mit dem Verfahren erhältlichen Nanopartikel, die insbesondere in Suspension vorliegen und gegen Agglomeration stabil sind, wobei die Suspension frei von organischen Kohlenstoffverbindungen ist, insbesondere kohlenwasserstofffrei, z.B. als einzige Kohlenstoffverbindung CO₂, insbesondere in Wasser gelöst, z.B. als Carbonatanionen, aufweist und besonders bevorzugt kohlenstofffrei ist. Insofern sind die erfindungsgemäß hergestellten Nanopartikel rein. In Suspension, die insbesondere eine wässrige Suspension ist, können die Nanopartikel in Form von Clustern vorliegen, die bevorzugt Vollschalencluster sind, insbesondere aus einer definierten Anzahl Atomen, z.B. Au-Cluster aus 55 Atomen. Die Nanopartikel können im Anschluß an ihre Herstellung optional aneinander angelagert sein.

Weiter betrifft die Erfindung eine Vorrichtung, die für das Herstellungsverfahren von Nanopartikeln angepasst ist.

Weiter betrifft die Erfindung Verbindungen und Beschichtungen der mit dem Verfahren erhältlichen Nanopartikel, beispielsweise mit den Nanopartikeln verbundene organische Moleküle, insbesondere biologisch aktive Verbindungen, sowie mit den Nanopartikeln beschichtete anorganische Träger, Elektroden oder optische Bauteile, insbesondere optische Elemente, die eine Schicht aus den Nanopartikeln aufweisen. Solche Verbindungen werden z.B. durch Kontaktieren der Nanopartikel, die zumindest frei von organischen Kohlenstoffverbindungen sind, mit einem organischen Molekül erhalten. Die Nanopartikel bestehen bevorzugt aus einem Edelmetall, insbesondere aus Gold und/oder einem Platinmetall, z.B. Platin.

### Stand der Technik

Die WO 2012/080458 A1 beschreibt ein Verfahren zur Herstellung metallischer Nanopartikel durch Laserablation, die anschließend mit wasserunlöslichen Mikropartikeln in Suspension gemischt werden, um die Nanopartikel auf den Mikropartikeln adsorbieren zu lassen. Bei dem Verfahren soll der Oxidationsgrad der Nanopartikel durch Einsatz eines organischen Lösungsmittels, insbesondere Aceton, Ethanol oder Isopropanol beeinflusst werden, oder durch Einsatz von Liganden, beispielsweise durch Natriumcitrat.

Die WO 2010/087869 A1 beschreibt die Herstellung von Nanopartikeln durch ultrakurze gepulste Laserablation in Flüssigkeiten, die sich dadurch auszeichnet, dass die Flüssigkeit, insbesondere Wasser, keine chemischen Stabilisatoren enthalten soll, wobei die Stabilität der Suspension der Nanopartikel durch Flüssigkeitsbewegung erhöht werden kann.

Die WO 2010/007117 beschreibt ein Verfahren zur Herstellung von Konjugaten aus metallischen Nanopartikeln mit einem organischen Bestandteil durch Bestrahlen eines Metallkörpers mit einem Laserstrahl, wobei ein den Metallkörper umgebendes Trägerfluid, das mit einer Vorläuferverbindung des organischen Bestandteils versetzt ist, bewegt wird.

Lopez-Sanchez et al, Nature Chemistry 1-6 (2011) beschreiben die Herstellung von metallischen Nanopartikeln in reduzierendem Medium mit Polyvinylalkohol (PVA) als Stabilisator, der von geträgerten Nanopartikeln durch Erwärmen auf bis zu 400°C oder Waschen mit heißem Wasser zumindest teilweise entfernt wurde.

### Aufgabe der Erfindung

Der Erfindung stellt sich die Aufgabe, ein alternatives Verfahren zur Herstellung von Nanopartikeln, die Cluster sein können, bereitzustellen, die bevorzugt in Medium ohne organische Kohlenstoffverbindung, z.B. in einem kohlenstofffreiem Medium wie Wasser eine stabile Suspension bilden. Eine weitere Aufgabe liegt in der Bereitstellung von Nanopartikeln, die Cluster sein können, insbesondere aus Gold, die eine homogene Partikelgrößenverteilung aufweisen, sowie Verbindungen, die die Nanopartikel aufweisen.

### Allgemeine Beschreibung der Erfindung

Die Erfindung löst die Aufgabe mit den Merkmalen der Ansprüche und stellt ein Verfahren zur Herstellung von Nanopartikeln bereit, die durch das Verfahren hergestellten besonderen Nanopartikel, sowie eine Vorrichtung, die zur Herstellung der Nanopartikel eingerichtet ist. Das Verfahren zeichnet sich insbesondere dadurch aus, dass die Nanopartikel rein, insbesondere frei von organischen Kohlenstoffverbindungen, bevorzugt kohlenstofffrei, und kontinuierlich erhalten werden. Das Verfahren kann vollständig oder teilweise kontinuierlich ausgeführt werden und ist daher auf einfache Weise skalierbar. Die Nanopartikel, die mit dem erfindungsgemäßen Verfahren erhältlich sind, zeichnen sich dadurch aus, dass sie ohne einen organischen Liganden in Suspension vorliegen und insbesondere bevorzugt als Suspension gegen Agglomeration stabil sind, wobei das Medium mit den darin suspendierten Partikeln frei von organischen Kohlenstoffverbindungen, insbesondere kohlenstofffrei ist. Generell werden unter den erfindungsgemäßen Nanopartikeln auch deren Cluster aus Metallatomen, insbesondere Vollschalencluster gefasst.

Das Verfahren zur Herstellung von Nanopartikeln sieht vor, mit einer ersten Laserstrahlung, die insbesondere gepulst, bevorzugt eine Kurzpulsstrahlung ist, aus einem metallhaltigen Körper erste Partikel zu erzeugen, wobei der metallhaltige Körper in einem wässrigen Medium angeordnet ist. Der metallhaltige Körper kann insbesondere ein Metallkörper (Oxidationsstufe 0) sein, wahlweise in Drahtform oder ein Pulver oder Mikropulver (Korngröße z.B. von 1 µm bis 500 µm) das in dem Medium suspendiert sein kann. Als Metall kann insbesondere ein Metall der Platingruppe, z.B. Platin, bevorzugt Gold enthalten sein; bevorzugt besteht der metallhaltige Körper aus Gold, bevorzugt mit einer Reinheit von größer als 99,9%, optional aus einer Legierung von zwei oder mehr Metallen der Platingruppe, z.B. AuPt.

Die erste Laserstrahlung ist vorzugsweise eine Kurzpulsstrahlung mit hoher Pulsenergie, vorzugsweise mit einer Pulsenergie von 1 bis 100 mJ, insbesondere mit Repetitionsraten von 10 Hz bis 500 kHz, insbesondere 0,1 bis 10 kHz, z.B. mit einer Pulsdauer von 100 ns, bevorzugt von maximal 10 ns, bevorzugter von maximal 10 ps, bei einer Wellenlänge von größer 330 nm und maximal 1500 nm, insbesondere im Bereich von 532 bis 1064 nm, bevorzugt 1064 nm. Die Zeitdauer der Bestrahlung des metallhaltigen Körpers beträgt vorzugsweise ca. 5 bis 10 min, z.B. 10 min je 100 mL Medium, bei einer Pulsenergie von ca. 80 mJ bei 10 ns Pulsdauer oder ca. 100 µJ bei 10 ps Pulsdauer.

Generell bevorzugt wird das Medium während der Bestrahlung des metallhaltigen Körpers mit der ersten Laserstrahlung bewegt, beispielsweise durch Rühren oder Überströmen des metallhaltigen Körpers mit dem Medium. Alternativ oder zusätzlich wird die erste Laserstrahlung relativ zum metallhaltigen Körper bewegt, z.B. durch Bewegen des Laserstrahls oder durch Bewegen des metallischen Körpers.

Erfindungsgemäß weist das Medium bevorzugt auf oder besteht aus Wasser, das bevorzugt frei ist von organischen Kohlenstoffverbindungen, insbesondere als einzige Kohlenstoffverbindung CO₂ enthält und bevorzugter kohlenstofffrei ist, optional mit einem Gehalt an zumindest einem anorganischen Oxidationsmittel. Das anorganische Oxidationsmittel kann z.B. ausgewählt sein aus H₂O₂, Ozon, Hypochlorid und Mischungen dieser.

Alternativ können erste Partikel dadurch erzeugt werden, dass organische Verbindungen von einem Präparat erster Partikel entfernt werden, wobei dieses Präparat z.B. durch einen Sol-Gel-Prozeß erhältlich ist und organische Verbindungen als Stabilisator enthält, z.B. PVA und/oder organisches Lösungsmittel. Bevorzugt sind organische Verbindungen in diesem Präparat an die ersten Partikel gebunden. Zur Entfernung organischer Verbindungen kann dieses Präparat erwärmt werden, z.B. auf 200°C bis 400°C oder darüber, vorzugsweise bis auf eine Temperatur unterhalb der Schmelztemperatur der ersten Partikel, und/oder mit Medium, das keine organischen Kohlenstoffverbindungen enthält, insbesondere kohlenstofffrei ist, gewaschen werden, z.B. zumindest 3-fach, und/oder oxidiert werden, z.B. durch Kontaktieren mit einem Oxidationsmittel, das organische Bestandteile zu CO₂ und nichtkohlenstoffhaltigen Produkten zersetzt. Alternativ zum Kontaktieren mit Oxidationsmittel kann das Präparat zur Entfernung organischer Kohlenstoffverbindungen einer Laserstrahlung ausgesetzt werden.

Organische Kohlenstoffverbindungen, z.B. Zitrat, Polyvinylpyrrolidon (PVP), PVA, Polyethylenglykol (PEG), Gerbsäure, und/oder anorganische Ionen, z.B. Alkali-, Erdalkaliiionen, Halogenionen und weitere anorganische Anionen, z.B. Phosphat, Sulfat, die in einem Präparat erster Partikel enthalten sind, können z.B. Synthesereste sein oder als Stabilisator der ersten Partikel wirken.

Das erfindungsgemäße Verfahren sieht vor, die durch das Aufbringen der ersten Laserstrahlung auf den metallhaltigen Körper erzeugten ersten Partikel, die in dem Medium enthalten sind, mit einer gepulsten Laserstrahlung zu bestrahlen, die auch als zweite Laserstrahlung bzw. als zweite gepulste Laserstrahlung bezeichnet wird, um die erfindungsgemäßen Nanopartikel zu erzeugen. Dabei wird ein Strom des Mediums, das frei ist von organischen Kohlenstoffverbindungen, insbesondere als einzige Kohlenstoffverbindung CO₂ enthält und bevorzugter kohlenstofffrei ist, wobei das Medium ein anorganisches Oxidationsmittel enthält, mit den darin suspendierten ersten Partikeln erzeugt und dieser Strom wird mit der zweiten gepulsten Laserstrahlung bestrahlt. Vorzugsweise ist der Strom des Mediums ein freier Strom, beispielsweise ein Strom, der ohne Kontakt mit einer festen Oberfläche zumindest in dem Abschnitt vorliegt, der von der zweiten gepulsten Laserstrahlung bestrahlt wird. Insbesondere ist der Strom des Mediums zumindest in dem von der zweiten gepulsten Laserstrahlung bestrahlten Abschnitt nicht in einem Rohr geführt, sondern z.B. in der Form als kontinuierlicher oder tropfenförmiger Flüssigkeitsstrom in einem gasgefüllten Raum. Der Strom des Mediums mit den darin suspendierten ersten Partikeln ermöglicht vorteilhafter Weise die Bestrahlung mit hoher Fluenz.

Generell können die ersten Partikel durch ein alternatives Verfahren hergestellt werden, das deren Herstellung mittels Bestrahlung eines metallhaltigen Körpers mit einer ersten Laserstrahlung ersetzt. Für die Zwecke der Erfindung kann auch für diese Ausführungsformen die Laserstrahlung, die auf die ersten Partikel gerichtet wird, als zweite Laserstrahlung bezeichnet werden. Insbesondere bei alternativen Ausführungsformen des Verfahrens kann das anorganische Oxidationsmittel vor oder nach dem Herstellen erster Partikel dem Medium zugesetzt werden, das die ersten Partikel enthält. Ein solches alternatives Verfahren zur Herstellung erster Partikel kann z.B. die Schritte der Drahterosion aufweisen, bei dem z.B. ein metallhaltiger Körper, insbesondere in Drahtform, z.B. ein Draht aus Gold und/oder einem Platinmetall in Wasser mit elektrischer Spannung beaufschlagt wird.

Das anorganische Oxidationsmittel kann im Medium vorgelegt sein, in dem der metallhaltige Körper während der Bestrahlung mit erster Laserstrahlung angeordnet ist und/oder während der Bestrahlung mit erster Laserstrahlung kontinuierlich oder satzweise zugegeben werden. Alternativ oder zusätzlich kann das anorganische Oxidationsmittel dem Medium vor Bestrahlen mit der zweiten Laserstrahlung kontinuierlich oder satzweise zugesetzt werden.

Optional kann das Medium anorganische Ionen aufweisen, z.B. Alkaliionen, Erdalkaliionen, Halogenionen und/oder anorganische Anionen, z.B. Phosphat, Sulfat; bevorzugt besteht das Medium aus Wasser und zumindest einem anorganischen Oxidationsmittel und ist frei von anorganischen Ionen.

In Ausführungsformen, in denen erste Partikel anorganische Ionen und/oder organische Verbindungen aufweisen, wie z.B. mit Bezug auf Präparate erster Partikel beschrieben, wird dem Medium bevorzugt anorganisches Oxidationsmittel in einer Menge zugesetzt, die zusätzlich zu einem oxidativen Milieu zur Oxidation der organischen Verbindungen ausreicht, wobei die zusätzliche Menge z.B. zumindest zur Oxidation zu CO₂ ausreicht.

Optional wird der pH-Wert des Mediums vor der Bestrahlung mit erster Laserstrahlung und/oder vor der Bestrahlung mit zweiter Laserstrahlung auf einen Wert von 3 bis 6,5 eingestellt.

Bevorzugt ist die zweite Laserstrahlung auf den Strom des Mediums mit den darin suspendierten ersten Partikeln fokussiert, wobei bevorzugt der Durchmesser des Stroms des Mediums mit den darin suspendierten ersten Partikeln maximal so groß ist, wie der Fokus der zweiten gepulsten Laserstrahlung. Bevorzugt liegt die Wellenlänge der zweiten Laserstrahlung in dem Bereich des Extinktionsmaximums der in dem Medium suspendierten ersten Partikel, z.B. im grünen Bereich, insbesondere bei 510 bis 540 nm.

Die zweite gepulste Laserstrahlung kann die Eigenschaften haben, wie sie mit Bezug auf die erste Laserstrahlung beschrieben sind, insbesondere bei einer Repetitionsrate von zumindest 10 Hz, bevorzugter von zumindest 100 Hz, bevorzugter von zumindest 1000 Hz, insbesondere für Pulsdauern von 1 bis 100 ns, bei 1 bis 500 kHz für Pulsdauern von 0,5 bis 100 ps, bevorzugt 1 bis 20 ps.

Ein Grund dafür, dass zumindest in dem Abschnitt, in dem das Medium mit den darin suspendierten ersten Partikeln von der zweiten gepulsten Laserstrahlung bestrahlt wird, bevorzugt in einem freien Strom geführt wird, ist die Vermeidung von Wechselwirkungen der Laserstrahlung und/oder der erzeugten Partikel mit einem Wandmaterial. Weiterhin sind in dieser Ausführungsform hohe Laserfluenzen, beispielsweise mit einer Laserfluenz von 0,1 bis 25 J/cm³, bevorzugt >1 J/cm² möglich, ohne dass eine entstehende Ultraschallwelle, Kavitationsblase, Stoßwelle, Laserabsorption oder thermische Effekte ein Wandmaterial, das den Strom des Mediums kontaktiert, insbesondere eine Kapillare, belasten würde. Dieses Verfahren und die dafür verwendete Vorrichtung erlauben eine intensive Bestrahlung der Partikel und insbesondere die Herstellung von Nanopartikeln mit einer engen Partikelgrößenverteilung, die für die Stabilität der Nanopartikel in Suspension vorteilhaft ist.

Das Medium, in dem der metallhaltige Körper während des Aufbringens der ersten gepulsten Laserstrahlung angeordnet ist, kann von dem Medium abweichen, aus dem ein Strom erzeugt wird, wobei in diesem Medium die ersten Partikel suspendiert sind. Z.B. kann das Medium, in dem der metallhaltige Körper während der Bestrahlung mit der ersten gepulsten Laserstrahlung angeordnet ist, ein wässriges Medium sein, insbesondere frei sein von einem anorganischen Oxidationsmittel, insbesondere aus reinem Wasser bestehen, während das Medium, aus dem der Strom erzeugt wird, von dem zumindest ein Abschnitt mit der zweiten gepulsten Laserstrahlung bestrahlt wird, das anorganische Oxidationsmittel enthält, insbesondere aus Wasser und zumindest einem anorganischen Oxidationsmittel besteht. Bevorzugt ist der pH-Wert und der Gehalt an gelöstem Gas des Mediums, in dem der metallhaltige Körper während des Aufbringens der ersten gepulsten Laserstrahlung angeordnet ist, eingestellt, z.B. durch Zugabe anorganischer Säure bzw. Base, bzw. durch Begasen oder Ausgasen.

Bevorzugt weist der Strom in dem Abschnitt, in dem er von der zweiten Laserstrahlung bestrahlt wird, einen Durchmesser von maximal 3 mm auf, insbesondere von 1 bis 3 mm, z. B. von 1,2 mm, wobei z.B. der zweite Laserstrahl einen Rohstrahldurchmesser von maximal 8 mm, bevorzugt maximal 6 mm aufweisen kann. Weiter bevorzugt ist der Strom vertikal nach unten gerichtet, bzw. ein frei vertikal fallender Strom des Mediums mit darin suspendierten ersten Partikeln. Bevorzugt ist der zweite Laserstrahl senkrecht auf den Strom dieses Mediums gerichtet.

Bevorzugt wird der Strom dadurch erzeugt, dass das Medium mit den darin suspendierten ersten Partikeln durch eine Düse austreten gelassen wird, deren Längsachse bevorzugt vertikal angeordnet ist. Die Düse ist bevorzugt kontinuierlich mit dem Medium mit den darin suspendierten ersten Partikeln beaufschlagt, beispielsweise aus einem Vorratsbehälter, bevorzugt unmittelbar im Anschluss an das Aufbringen der ersten gepulsten Laserstrahlung auf den ersten metallhaltigen Körper, sodass das Medium mit den darin suspendierten ersten Partikeln bevorzugt innerhalb von 60 min, bevorzugter innerhalb von 10 min, bevorzugter innerhalb 1 min zum Erzeugen des Stroms verwendet wird, beispielsweise durch Zuführung zu einer Düse.

In Alternative zum freien Strom kann das Medium, das die ersten Partikel enthält, während es mit der gepulsten Laserstrahlung bestrahlt wird, in einem Behälter enthalten sein, ohne oder mit Flüssigkeitsbewegung. Ein solcher Behälter kann geschlossen oder offen sein und das Medium mit den ersten Partikeln enthalten oder von dem Medium mit den ersten Partikeln durchströmt werden. Ein Behälter kann z.B. eine Durchflusskammer bzw. ein Rohr sein, wobei der gepulste Laser einen Volumenabschnitt durchstrahlt, wobei der Laser insbesondere eingerichtet ist, jedes Volumenelement des Mediums mit den ersten Partikeln zumindest oder genau einmal zu durchstrahlen.

Weiter bevorzugt wird dem Medium nach Bestrahlung mit dem zweiten Laserstrahl anorganisches Oxidationsmittel zugesetzt, z.B. in Abhängigkeit von der Konzentration und/oder Größe erzeugter Nanopartikel gesteuert zugesetzt. Das im Anschluß zugesetzte anorganische Oxidationsmittel kann dasjenige sein, das das Medium vor Bestrahlung mit dem zweiten Laserstrahl enthält, oder ein anderes anorganisches Oxidationsmittel.

Die mit dem Verfahren erzeugten Nanopartikel weisen bevorzugt eine maximale Größe von etwa 5 nm auf, insbesondere mit einer mittleren Größe von 2 bis 3 nm, insbesondere von 2,5 nm. Bevorzugt weisen zumindest 50%, bevorzugter zumindest 75%, insbesondere bevorzugt zumindest 90% der Nanopartikel eine solche mittlere Größe auf. Bei Vorliegen dieser Nanopartikel in einem Medium in Suspension, die aus dem Medium und diesen Nanopartikeln besteht und daher insbesondere keine weiteren Partikel, z.B. keine ersten Partikel enthält, sind die Nanopartikel bevorzugt nicht oberflächenplasmonresonant, z.B. Nanopartikel aus Gold nicht oberflächenplasmonresonant bei Bestrahlung mit einer Wellenlänge von 520 nm. Nanopartikel, die Cluster sind, können Vollschalencluster wie M₅₅, M₃₀₉, M₅₆₁, insbesondere Au₅₅ oder Pt₃₀₉ sein.

Es hat sich gezeigt, dass Bestrahlen des Stroms aus Medium, dem ein anorganisches Oxidationsmittel zugesetzt ist, mit darin suspendierten ersten Partikeln mit einer gepulsten Laserstrahlung, die für die Zwecke der Erfindung auch als zweite gepulste Laserstrahlung bezeichnet wird, Nanopartikel dieser Größe erzeugt.

Überraschenderweise hat sich gezeigt, dass die Anwesenheit eines anorganischen Oxidationsmittels in dem Medium, das erste, durch Laserbestrahlung eines metallhaltigen Körpers erzeugte Partikel enthält, zur Stabilität der durch Bestrahlen des Stroms mit einer zweiten gepulsten Laserstrahlung zur Erzeugung einer stabilen Zusammensetzung bzw. stabilen Verteilung der Nanopartikel in dem Medium führt. Entsprechend liegt ein besonderer Vorteil des erfindungsgemäßen Verfahrens und der damit erhältlichen Nanopartikel darin, dass die Zusammensetzung bzw. das Medium, in dem die Nanopartikel enthalten sind, kein Stabilisationsmittel, insbesondere keine organischen Verbindungen zu enthalten braucht. Die Stabilität der Suspension der Nanopartikel gegen Anlagerung wird gegenwärtig auf die zumindest teilweise oberflächliche, bevorzugt vollständige oberflächliche Oxidation der Nanopartikel zurückgeführt, bzw. darauf, dass die Nanopartikel in der Suspension elektrostatisch stabilisiert sind.

Dadurch, dass die erzeugten Nanopartikel ohne organische Kohlenstoffverbindung bzw. kohlenstofffrei in dem Medium stabil sind, können sie ohne Abtrennung einer Kohlenstoffverbindung mit einer zugesetzten Substanz reagieren und beispielsweise eine Komplexverbindung eingehen, chemisorbieren oder eine Beschichtung bilden, die insbesondere aus den Nanopartikeln besteht, insbesondere frei von Kohlenstoff ist. Solche Komplexverbindungen mit Nanopartikeln oder von organischen Kohlenstoffverbindungen freie Beschichtungen aus Nanopartikeln, insbesondere kohlenstofffreie Beschichtungen aus Nanopartikeln weisen eine signifikante katalytische Aktivität auf. Für die Beschichtungen aus Nanopartikeln wird die katalytische Aktivität auf die große Oberfläche der Nanopartikel und/oder auf die Interaktion der Nanopartikel mit dem Substrat zurückgeführt und darauf, dass die Oberfläche der Nanopartikel nicht von Kohlenstoffverbindungen belegt ist.

Entsprechend enthält das Verfahren bevorzugt den Schritt, anschließend die Nanopartikel in dem Medium mit einer Substanz zu kontaktieren, die beispielsweise ein biologisches Molekül ist, insbesondere ausgewählt aus Nukleinsäure, beispielsweise einem Oligonukleotid, einem Protein, Peptid, einem Glycosid und/oder einem Lipid. Bevorzugt ist das Protein, das mit den Nanopartikeln kontaktiert ist und eine Verbindung damit bildet ein spezifisches Bindemolekül, z.B. ein Antikörper. Das biologische Molekül weist vorzugsweise eine Thiolgruppe oder Disulfidgruppe auf. Alternativ oder zusätzlich kann die Substanz ein Träger, insbesondere ein anorganischer Träger sein, sodass die Nanopartikel auf diesem Träger abgeschieden werden und den Träger beschichten. Dazu ist bevorzugt, dass die Nanopartikel elektrophoretisch auf dem anorganischen Träger abgeschieden werden, um eine oberflächliche Beschichtung zu bilden. Ein solcher anorganischer Träger ist insbesondere ein Katalysatorträger, beispielsweise ein Titanoxid, sodass beispielsweise die Nanopartikel eine katalytisch aktive Schicht bilden, oder der anorganische Träger kann eine Elektrode sein, auf dem die abgeschiedenen Nanopartikel eine Beschichtung bilden. Eine Elektrode, die eine Beschichtung aus den Nanopartikeln aufweist, ist insbesondere zur Verwendung als elektrophysiologische Elektrode geeignet. In diesen Ausführungsformen liegt ein Vorteil der erfindungsgemäßen Nanopartikel darin, dass diese ein Fluoreszenzsignal bei Bestrahlung mit einer Anregungswellenlänge erzeugen, dass von einer an die Nanopartikel gebundenen Substanz beeinflusst ist, bzw. für die gebundene Substanz spezifisch ist.

Bevorzugt werden die Nanopartikel direkt elektrochemisch mit dem Träger gekoppelt, z.B. durch Chemi- oder Physisorption. Eine direkte elektrochemische Kopplung der Nanopartikel mit einer Substanz, die insbesondere ein biologisches Molekül ist, oder mit einem Träger erlaubt eine Orbitalkopplung, die in Elektrochemolumineszenz oder Fluoreszenz resultieren. Die Abwesenheit von organischen Kohlenstoffverbindungen, bevorzugt von Kohlenstoff, von den eingesetzten Nanopartikeln weist eine mit dem Nanopartikel kontaktierte bzw. gebundene Substanz keine weiteren organischen Kohlenstoffverbindungen auf bzw. eine Beschichtung aus den Nanopartikeln weist keine organischen Kohlenstoffverbindungen, die die Wechselwirkung der Nanopartikel mit der Substanz bzw. dem Träger beeinflussen oder die die Reaktion der am Nanopartikel gebundenen Substanz oder der Beschichtung auf dem Träger beeinflussen. Daher können aus den erfindungsgemäßen Nanopartikeln Verbindungen mit Substanzen oder Beschichtungen auf Trägern erzeugt werden, deren Eigenschaften, z.B. Reaktions- oder Bindeeigenschaften, nicht durch organische Kohlenstoffverbindungen beeinträchtigt werden.

Weiterhin kann die Substanz bzw. der Träger ein optisch aktiver Träger sein, beispielsweise ein optisch transparenter Träger, insbesondere aus Glas oder Kunststoff, sodass die Abscheidung der erfindungsgemäß hergestellten Nanopartikel auf dem Träger zur optisch aktiven Beschichtung führt. In dieser Ausführungsform führt das Verfahren zur Herstellung eines optischen Elements, da die Beschichtung der erfindungsgemäß hergestellten Nanopartikel auf dem Träger eine optisch aktive Schicht ist, die z.B. bei Lichteinfall einer vorbestimmten Wellenlänge die Lichtdurchlässigkeit der Beschichtung und damit des optischen Elements bei Erreichen oder Überschreiten einer bestimmten eingestrahlten Fluenzintensität schnell verringert, insbesondere um zumindest 50%, bevorzugter um zumindest 90%, bevorzugter um zumindest 99% zu reduzieren. Ein solches optisches Element kann z.B. als optischer Schalter, insbesondere für Laserstrahlung, die optional gepulst sein kann, insbesondere mit einer Wellenlänge von 350-1064 nm dienen, oder für sichtbares Licht. Ein solches optisches Element kann wegen der sehr schnellen Verringerung einfallender Strahlung als optische Schutzeinrichtung verwendet werden.

Die Verfahren, die einen Schritt des Kontaktierens der Nanopartikel mit einer Substanz, z.B. einem biologischen Molekül oder einem Träger enthalten, weisen vorteilhafter Weise keinen vorherigen Schritt zur Entfernung einer organischen Verbindung aus der Suspension der Nanopartikel auf. So können bei erfindungsgemäßen Verfahren Nanopartikel auf Trägern z.B. ohne chemische oder thermische Schritte, insbesondere ohne das Kalzinieren angeordnet werden, so dass die Schritte, die z.B. bei einem Gehalt eines Nanopartikelpräparats an Kohlenstoff zur Entfernung organischer Verbindungen notwendig sind, erfindungsgemäß nicht erforderlich sind.

H₂O₂ oder Ozon als anorganisches Oxidationsmittel sind dadurch vorteilhaft, dass sie durch Bestrahlung mit Licht, durch Druckabsenkung und/oder Temperaturerhöhung aus Wasser, das das Medium bildet, entfernt werden können. Entsprechend kann das Verfahren optional den Schritt aufweisen, im Anschluss an die Bestrahlung des Stroms des Mediums mit den darin suspendierten ersten Partikeln, wobei das Medium das anorganische Oxidationsmittel enthält, das Medium mit den darin enthaltenen Nanopartikeln zu bestrahlen, um insbesondere das anorganische Oxidationsmittel, das aus H₂O₂ oder Ozon besteht, zumindest anteilig zu entfernen. Bevorzugt wird das Medium dabei unter Unterdruck gesetzt und/oder mit Strahlung einer Wellenlänge bestrahlt, bei der das anorganische Oxidationsmittel zersetzt wird, z.B. im UV-Bereich, sodass das anorganische Oxidationsmittel bzw. dessen Zersetzungsprodukte gasförmig austreten können.

Die Vorrichtung ist insbesondere zur Durchführung der Schritte des Verfahrens eingerichtet.

### Genaue Beschreibung der Erfindung

Die Erfindung wird nun genauer anhand von Beispielen und mit Bezug auf die Figuren beschrieben, die in
Fig. 1 schematisch den Aufbau einer erfindungsgemäßen Vorrichtung,
Fig. 2 ein Photospektrum erfindungsgemäß hergestellter Nanopartikel in Wasser mit H₂O₂,
Fig. 3a eine TEM-Aufnahme erfindungsgemäßer Nanopartikel,
Fig. 3b die per TEM gemessene Größenverteilung erfindungsgemäß hergestellter Nanopartikel,
Fig. 3c eine graphische Darstellung der mit den Daten aus Fig. 3b bestimmten Spärizität erfindungsgemäßer Nanopartikel,
Fig. 3d eine graphische Darstellung der Summenverteilung der Größen erfindungsgemäßer Nanopartikel,
Fig. 3e eine graphische Darstellung der per Scheibenzentrifuge gemessenen Größen erfindungsgemäßer Nanopartikel,
Fig. 4 eine rasterelektronenmikroskopische Aufnahme eines Trägers,
Fig. 5 eine rasterelektronenmikroskopische Aufnahme eines Trägers mit darauf angeordneten erfindungsgemäßen Nanopartikeln,
Fig. 6 eine rasterelektronenmikroskopische Aufnahme eines Trägers mit darauf angeordneten erfindungsgemäßen Nanopartikeln und
Fig. 7 das Ergebnis einer EDX-Analyse erfindungsgemäßer Nanopartikel auf einem Träger zeigen.

### Beispiel 1: Herstellung von Nanopartikeln aus Gold

Als metallhaltiger Körper wurde 99,99 %-iges Gold in hochreinem Wasser mit einem Gehalt an 10 Gew.-% H₂O₂ angeordnet und 10 ns Laserimpulsen bei einer Wellenlänge von 1064 nm mit einer maximalen Energie von 80 mJ pro Puls, Strahldurchmesser 6 mm bei einem Abstand von etwa 100 mm von der Linse (Brennweite 100 mm) und Metallkörper bei einer Pulswiederholungsrate von 100 Hz bestrahlt. Die Schichthöhe des Mediums zwischen Metallkörper und Laser betrug etwa 1 cm, der pH-Wert des Wassers ca. 3 bis 5.

Es konnte beobachtet werden, dass sich am Metallkörper eine Rotfärbung in der Flüssigkeit ausbreitete, wie dies für Goldnanopartikel bekannt ist. Es zeigte sich eine Plasmonresonanz (erhöhte Extinktion) bei 520 nm. Diese ersten Partikel agglomerierten zu hydrodynamischen Durchmessern von ca. 50 nm, gemessen durch dynamische Laserlichtstreuung und Messungen in einer Scheibenzentrifuge.

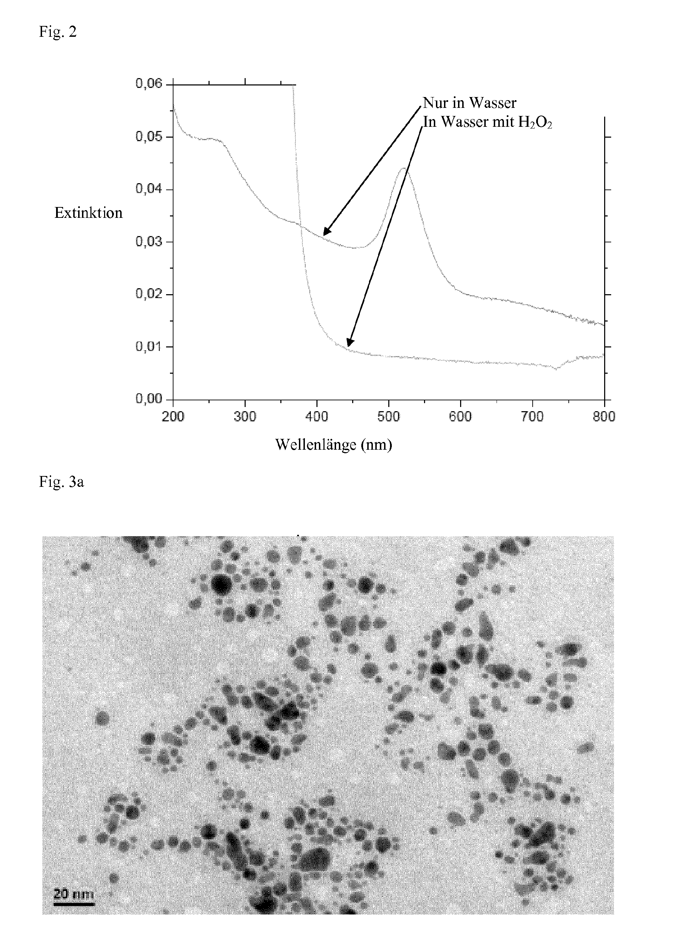

Das Medium mit den so hergestellten ersten Partikeln wurde unter Schwerkrafteinfluss oder mittels einer Pumpe durch eine Düse austreten gelassen, die innerhalb eines größeren Glaskörpers als Schutzbehälter angeordnet war. Die Düse war vertikal nach unten gerichtet und das Medium mit den darin suspendierten ersten Partikeln erzeugte einen frei fallenden Strom von ca. 1,2 mm Durchmesser. Ein zweiter Laser, der dieselben Strahleigenschaften wie der zuvor eingesetzte Laser jedoch die frequenzverdoppelte Wellenlänge von 532 nm hatte, wurde auf den Strom des Mediums fokussiert, sodass der fokussierte Laser den Strom des Mediums vollständig in einem Abschnitt erfasste. Die Geschwindigkeit des Stroms betrug ca. 0,6 m/s und die Volumenwechselrate des bestrahlten Abschnitts betrug 100 Hz. Die erhaltenen Nanopartikel zeigten eine Größe von kleiner etwa 5 nm, eine mittlere Anzahlgrößenverteilung von etwa 2,5 nm und zeigten generell keine Resonanzfrequenz im grünen Bereich, insbesondere keine bei 520 nm, wie dies im Spektrum von Figur 2 gezeigt ist. Die Rotfärbung und Extinktion bei 520 nm der ersten Partikel verschwand vollständig, wenn das Medium wiederholt durch den zweiten Laserstrahl hindurchtrat, z.B. bis zu 60 mal.

In bevorzugter Ausführung wurden die Nanopartikel, die durch Bestrahlen des Stroms des Mediums mit darin suspendierten ersten Partikeln erzeugt wurden, zumindest noch einmal in einen freien Strom von Medium mit der zweiten gepulsten Laserstrahlung bestrahlt, beispielsweise durch Rückführen der in dem Medium suspendierten Nanopartikel durch die Düse, wiederum mit abschnittsweiser Bestrahlung des Stroms mit der zweiten gepulsten Laserstrahlung. Wie generell bevorzugt wurde die Repetitionsrate des Lasers an die Geschwindigkeit des Stroms angepasst, dass jedes Volumenelement des Stroms genau einmal mit einem Puls der Laserbestrahlung beaufschlagt wird.

Die erhaltenen Nanopartikel waren ohne weiteren Zusatz in dem Medium, das anorganisches Oxidationsmittel enthielt, als Suspension stabil, z.B. für zumindest 4 d, bevorzugt für zumindest 5 bis 30 d. Die Stabilität konnte auch daran gesehen werden, dass keine Plasmonresonanz auftrat, die ein Anzeichen für eine Aneinanderlagerung von nicht plasmonenresonanten Nanopartikeln ist.

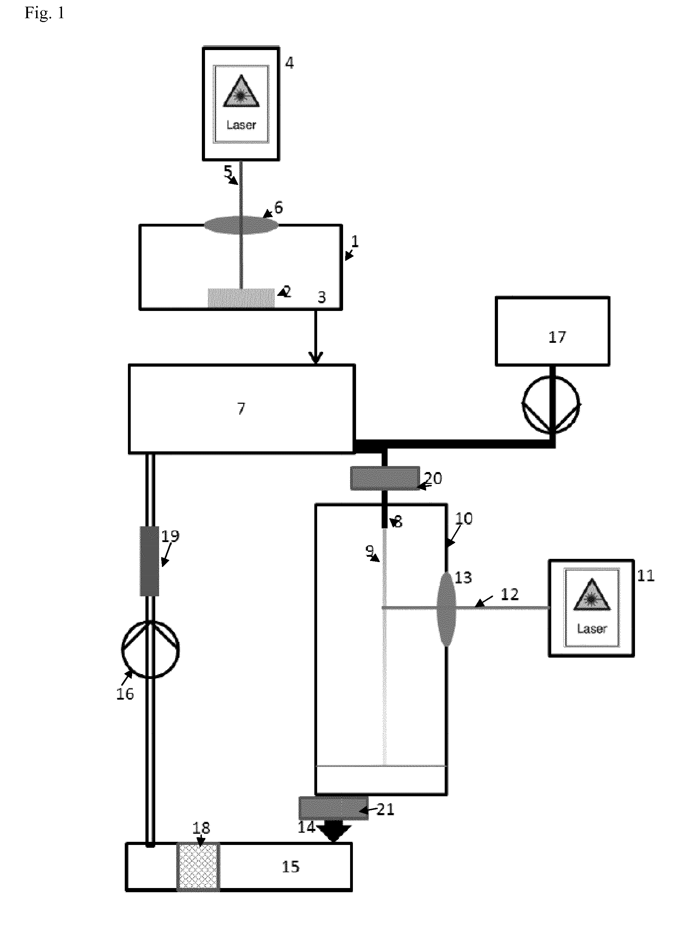

Die für dieses Verfahren eingesetzte Vorrichtung ist schematisch in der Figur 1 gezeigt. Dabei enthält ein Behälter 1 den Metallkörper 2 aus Gold auf einer Halteeinrichtung und ist von Medium 3 umgeben. Ein erster Laser 4 erzeugt die erste Laserstrahlung 5, die durch ein optisch transparentes Fenster 6 des Behälters 1 auf den Metallkörper 2 gerichtet ist. Bevorzugt ist der Behälter 1 eine von Medium 3 durchströmte Kammer mit einem Einlass und einem Auslass. Von dem Behälter 1 wird das Medium 3 im Anschluss an die Bestrahlung mit erster Laserstrahlung 5 in einen Vorratsbehälter 7 überführt, optional kontinuierlich. Optional ist in der Leitung zwischen dem Behälter 1 und dem Vorratsbehälter 7 und/oder in der Leitung zwischen dem Vorratsbehälter 7 und der Düse 8 ein Spektrometer 20 angeordnet, das zur Messung der Extinktion eingerichtet ist. Durch diese Anordnung ist das Spektrometer 20 in einer Leitung angeordnet, die vor der Düse 8 liegt und ist daher eingerichtet, das der Düse 8 zugeführte Medium zu messen. Bevorzugt ist ein Spektrometer 20 eingerichtet, eine Dosiereinrichtung für die Zugabe des anorganischen Oxidationsmittels zu steuern. Aus dem Vorratsbehälter 7 wird das Medium 3 mit den darin enthaltenen ersten Partikeln einer Düse 8 zugeleitet, die vertikal nach unten gerichtet ist und einen freien Mediumstrom 9 erzeugt. Zur Vermeidung von Aerosolbildung ist die Düse 8 innerhalb eines Gehäuses 10 angeordnet, in dem der Mediumstrom 9 frei fällt, optional durch eine Pumpe getrieben. Ein Abschnitt des Stroms des Mediums 9 wird von der von einem zweiten Laser 11 erzeugten zweiten Laserstrahlung 12 bestrahlt, die z.B. durch ein für die zweite Laserstrahlung 12 durchlässiges Fenster oder Loch 13 des Gehäuses 10 tritt. Wie schematisch angedeutet kann das Fenster 13 ein optisches Element, insbesondere eine Sammellinse aufweisen oder daraus bestehen. Das Gehäuse 10 weist einen Auslass 14 auf, aus dem das Medium mit den nun darin enthaltenen Nanopartikeln austritt, beispielsweise in ein Sammelgefäß 15. Optional ist ein weiteres Spektrometer 21 am Auslass 14 angeordnet, das insbesondere mit einer Steuerungseinrichtung des zweiten Lasers 11 oder mit einer Steuerungseinrichtung der Pumpe, die den Mediumstrom 9 treibt, verbunden sein kann. Das Sammelgefäß 15 kann mit einer Rückführleitung 16 mit der Düse 8 verbunden sein, um eine wiederholte Bestrahlung des Mediums mit den nun darin enthaltenen Nanopartikeln und/oder ersten Partikeln durch zweite Laserstrahlung 12 zu erlauben. Im Sammelgefäß 15 kann ein Träger 18 angeordnet sein, wobei der Träger 18 insbesondere den Strömungsquerschnitt des Sammelgefäßes 18 überdeckt. Ein solcher Träger 18 ist z.B. im Sammelgefäß 15 angeordnet, um Nanopartikel auf dem Träger 18 anzuordnen. Der Träger 18 kann z.B. Titandioxid oder Aluminiumoxid, als Pulver oder Formkörper sein, auf den Nanopartikel sorbieren. In der Rückführleitung 16, die eine Pumpe aufweisen kann, ist optional ein Filter 19 enthalten, der Partikel mit einer Größe oberhalb einer vorgegebenen Größe zurückhält, z.B. Partikel mit einer Größe oberhalb 10 nm zurückhält. Eine Dosiereinrichtung für anorganisches Oxidationsmittel ist anhand eines Vorratsbehälters 17 für das Oxidationsmittel gezeigt, der mittels einer Leitung mit der Leitung verbunden ist, die zur Düse 8 führt. Auf diese Weise kann die Dosiereinrichtung, die vorzugsweise abhängig von dem am Auslass 14 angeordneten Spektrometer 21 gesteuert ist, eingerichtet sein, dem Medium, das erste Partikel enthält, anorganisches Oxidationsmittel zuzuführen.

Figur 2 zeigt ein Spektrum von erfindungsgemäß hergestellten Nanopartikeln aus Gold in H₂O₂ -haltigem Wasser im Vergleich zu Nanopartikeln aus Gold in reinem Wasser und daher ohne anorganisches Oxidationsmittel, die ansonsten mit dem gleichen Verfahren erzeugt wurden. Die Abwesenheit der Plasmonresonanz bei 520 nm erfindungsgemäßer Nanopartikel zeigt, dass die erfindungsgemäß in dem Medium mit einen Gehalt an anorganischen Oxidationsmittel hergestellten Nanopartikel kleiner als 5 nm, insbesondere kleiner als 3,5 nm im Durchmesser sind und sich nicht zu größeren, plasmonresonanten Aggregaten aneinander angelagert haben. Die erfindungsgemäßen Partikel zeigen dieselben optischen Eigenschaften auch nach einer Lagerung von z.B. 20 Tagen und belegen daher die Stabilität der Nanopartikel, die in dem Medium suspendiert sind. Als Grund für diese Stabilität wird gegenwärtig eine elektrostatische Stabilisierung der Nanopartikel angenommen. Daher kann die Suspension der Nanopartikel, die mit dem erfindungsgemäßen Verfahren erhältlich ist, auch als elektrostatisch stabilisiertes Kolloid bezeichnet werden.

Figur 3a zeigt die Partikelgrößen der erfindungsgemäß hergestellten Nanopartikel aus Gold in einer TEM-Aufnahme, die auf einem Gitter abgelagert und im Transmissionselektronenmikroskop (TEM) vermessen wurden. Diese Daten zeigen, dass die erfindungsgemäß hergestellten Nanopartikel eine mittlere Partikelgröße von ca. 3 nm bzw. einen Größenbereich von ca. 1-5 nm aufweisen können.

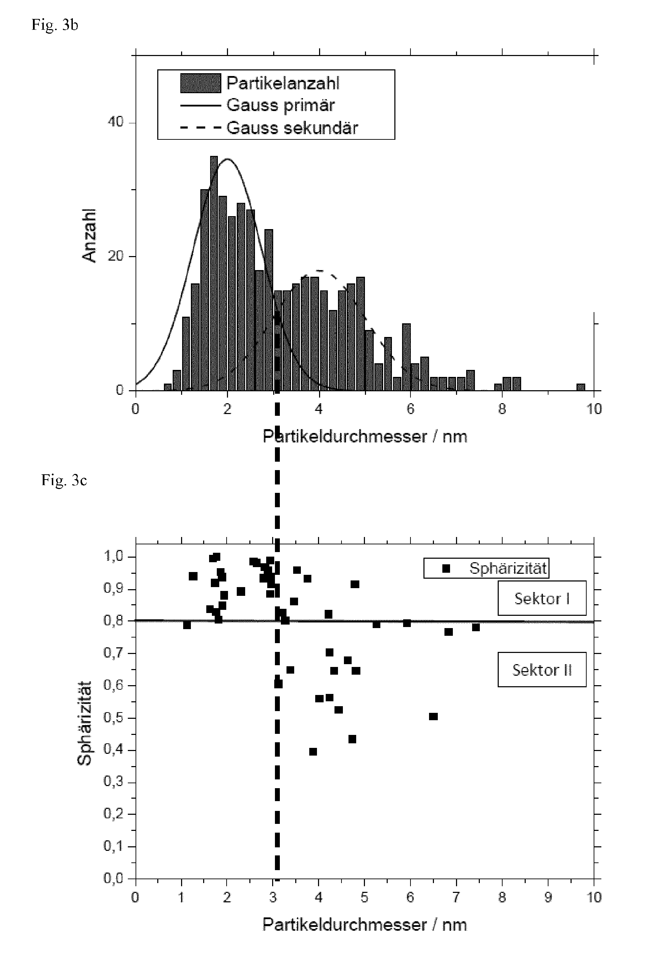

Figur 3b zeigt eine Größenverteilung erfindungsgemäßer Nanopartikel, die aus TEM-Aufnahmen bestimmt wurde. In der Größenverteilung sind eine primäre und eine sekundäre Gaußverteilung bestimmt. Die Partikelgröße, die im Schnittpunkt der Gaußverteilungen liegt, ist als Grenze bei der Darstellung der Sphärizität in Fig. 3c gezeigt. Für eine Sphärizität von unterhalb 0,8 (Sektor II) wird keine Kugelform angenommen.

Aus Figuren 3b und 3c ergibt sich, dass die kugelförmigen Partikel in Sektor I erfindungsgemäße Nanopartikel sind, die eine Partikelgröße von kleiner als 5 nm aufweisen, wobei ihr Hauptanteil eine Größe von kleiner 3 nm hat.

Aus den Daten der Figuren 3b und 3c kann geschlossen werden, dass größere Partikel möglicherweise erste Partikel sind oder als Artefakte bei der Präparation der Partikel auf dem TEM-Gitter entstanden sind, z.B. verursacht durch Entfernen des anorganischen Oxidationsmittels.

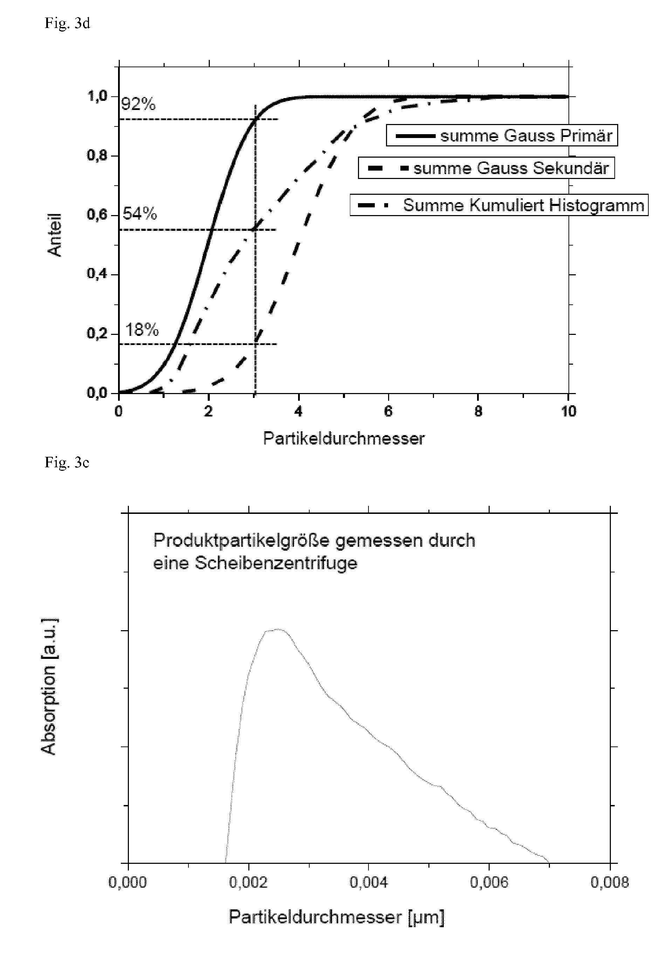

Figur 3d zeigt die Summenverteilung der einzelnen und kumulierten Gauß-Verteilungen. Hieraus wird ersichtlich, dass vorzugsweise 92% aller nach dem Verfahren erzeugen Partikel in dem Medium mit einem Durchmesser kleiner als 3 nm vorliegen..

Die in Figur 3e gezeigten Daten der Messung der Größe der Nanopartikel mittels einer Scheibenzentrifuge. Diese Daten bestätigen, dass erfindungsgemäße Nanopartikel eine Größe von im Wesentlichen kleiner als 5 nm aufweisen, mit einer mittleren Größe von kleiner 3 bis kleiner 4 nm.

### Beispiel 2: Herstellung einer Verbindung aus Nanopartikeln mit organischen Liganden

Nach Beispiel 1 erzeugte Nanopartikel aus Gold in einem Medium mit einem Gehalt an H₂O₂ wurden zunächst in ein nicht oxidierendes Medium überführt. Anschließend wurde die Suspension der Nanopartikel mit einem organischen Liganden als Beispiel für eine Substanz kontaktiert, beispielsweise mit einem Oligonukleotid, einem Protein, bevorzugt einem Bindemolekül, insbesondere einem Antikörper, oder mit einem Polysaccharid. Optional wies die Substanz eine Thiolgruppe auf.

Es hat sich gezeigt, dass die erfindungsgemäß erhältlichen Nanopartikel eine ausreichende Reaktivität aufweisen, um mit dem als Substanz zugesetzten organischen Molekül eine Bindung einzugehen. Die organischen Moleküle waren daher durch die Nanopartikel markiert. Die erhaltenen Verbindungen aus der Substanz mit erfindungsgemäß hergestellten Nanopartikeln waren fluoreszent und nicht plasmonresonant, wie dies generell bevorzugt ist.

### Beispiel 3: Beschichten eines anorganischen Trägers mit Nanopartikeln

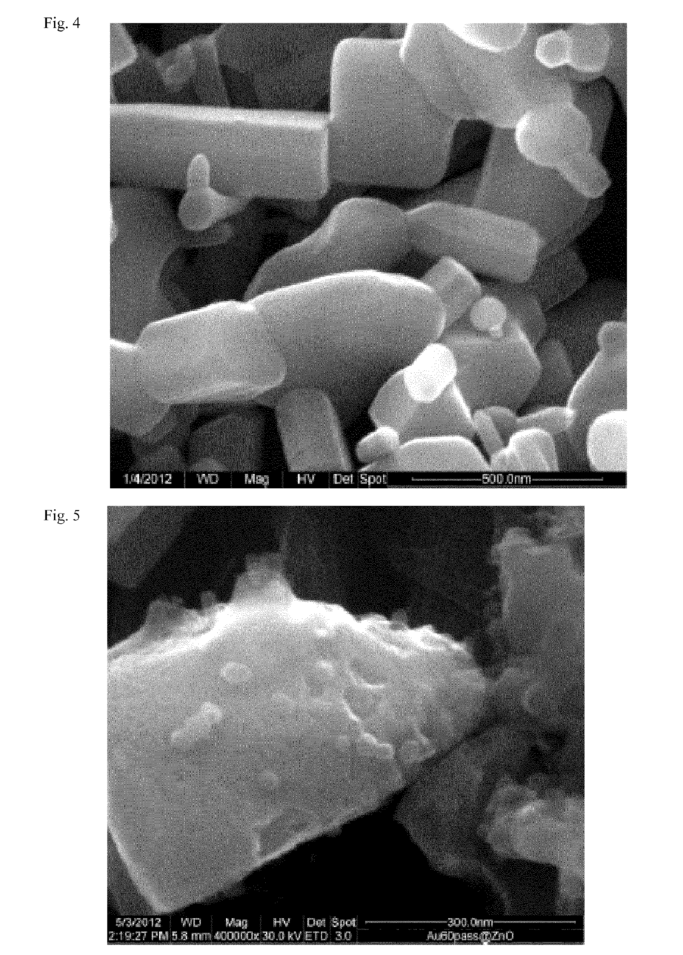

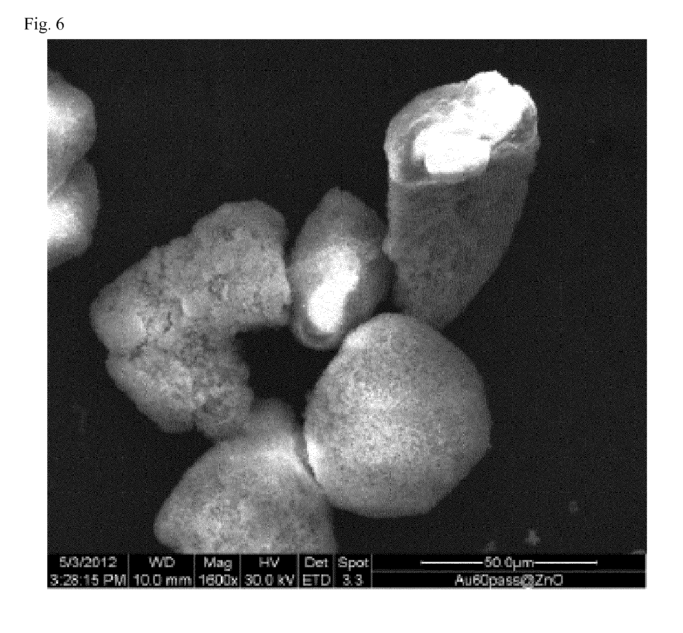

Als Beispiel für einen anorganischen Träger wurde Zinkoxid eingesetzt oder eine Elektrode mit metallischer Oberfläche. Die Nanopartikel wurden durch Kontaktierung mit dem Medium, das die Nanopartikel enthielt, auf dem Träger abgeschieden. Dabei zeigte sich, dass kein äußeres oder zusätzliches elektrisches Feld zur Anordnung der Nanopartikel auf dem Träger erforderlich war. Die Nanopartikel konnten alternativ durch elektrophoretische Abscheidung oberflächlich auf den Träger, der bevorzugt Oberflächenladungen aufwies, abgeschieden werden und bildeten eine adsorbierte Schicht. Das Zinkoxid, das mit den Nanopartikeln aus Gold beschichtet war, zeichnete sich durch eine homogene Anordnung bzw. Schicht aus sorbierten Nanopartikeln aus. Figur 4 zeigt die als Träger eingesetzten Zinkoxidpartikel in einer rasterelektronenmikroskopischen (REM) Aufnahme, die Figuren 5 und 6 zeigen die Zinkoxidpartikel nach Kontaktierung mit den suspendierten Nanopartikeln. Hier wird deutlich, dass diese Nanopartikel auf der Oberfläche des Trägers sorbieren.

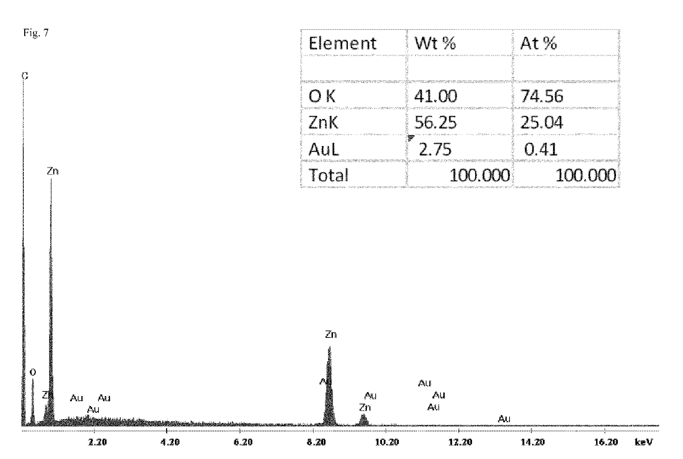

Figur 7 zeigt eine EDX-Analyse der erfindungsgemäßen Nanopartikel aus Gold, die auf ZnO-Träger angeordnet sind. Sauerstoff ist anhand der K-Linie (O K), Zink anhand der K-Linie (ZnK) und Gold anhand der L-Linie (AuL) des Spektrums bestimmt worden.

Aus der EDX-Analyse wird deutlich, dass die Nanopartikel aus Gold, die nichtplasmonresonant sind und die bevorzugt Cluster sind, auf den Träger sorbiert sind und eine nicht-plasmonresonante Schicht bilden.

### Beispiel 4: Herstellung eines optischen Elements

Als Beispiel für ein optisches Element wurde ein Glas mit den in dem Medium suspendierten Nanopartikeln kontaktiert. Das Medium konnte den Gehalt an anorganischen Oxidationsmittel aufweisen, alternativ konnte das anorganische Oxidationsmittel aus dem Medium entfernt werden.

Die erfindungsgemäß erhältlichen Nanopartikel konnten durch bloßes Kontaktieren des Glases mit der Suspension darauf abgeschieden werden.

Das so hergestellte optische Element zeigte bei Bestrahlung, insbesondere in einem Wellenlängenbereich von 350 bis 1064 nm eine limitierbare Lichtundurchlässigkeit, die die Eignung der erfindungsgemäß erhältlichen Nanopartikel zur Verwendung als optische Begrenzer gegen Strahlung dieser Wellenlänge zeigen.

### Beispiel 5: Herstellung von Nanopartikeln aus Gold

Entsprechend Beispiel 1 wurden erste Partikel mit gepulster Laserstrahlung bestrahlt, wobei die ersten Partikel nicht durch Bestrahlen des Metallkörpers mit erster Laserstrahlung erzeugt wurden, sondern kolloidchemisch synthetisiert und in wässrigem Medium vorlagen. Diese ersten Partikel wiesen zur Stabilisierung PVA auf.

Zur Entfernung der organischen Kohlenstoffverbindung PVA wurde das Medium, in dem die ersten Partikel enthalten waren, vor Bestrahlung mit gepulster Laserstrahlung mit Reinstwasser zumindest 3-fach gewaschen und in Reinstwasser resuspendiert, mit H₂O₂ in zusätzlicher Menge versetzt, die stöchiometrisch zur vollständigen Oxidation der organischen Kohlenstoffverbindung ausreichte, und/oder zusätzlich mit einem gepulsten Laser bestrahlt, bevor H₂O₂ zugesetzt wurde.

Die Bestrahlung der ersten Partikel wurde bevorzugt im freien Flüssigkeitsstrom durchgeführt, alternativ in einem Behälter unter Rühren.

## Patentansprüche

1. Verfahren zur Herstellung von Nanopartikeln aus Gold und/oder einem Platinmetall durch Bereitstellen erster Partikel aus Gold und/oder dem Platinmetall, die in einem wässrigen Medium suspendiert sind, **gekennzeichnet dadurch, dass** dem wässrigen Medium ein anorganisches Oxidationsmittel zugesetzt ist und das Medium mit einer gepulsten Laserstrahlung bestrahlt wird, wodurch eine Suspension der Nanopartikel in dem Medium erzeugt wird.

2. Verfahren nach einem der voranstehenden Ansprüche, **dadurch gekennzeichnet, dass** das wässrige Medium und die darin suspendierten ersten Partikel von organischen Kohlenstoffverbindungen frei sind.

3. Verfahren nach Anspruch 1 oder 2, **dadurch gekennzeichnet, dass** das wässrige Medium mit den darin suspendierten ersten Partikeln hergestellt ist aus ersten Partikeln, die in Mischung mit organischen Verbindungen vorliegen, durch Entfernen oder Oxidieren der organischen Verbindungen.

4. Verfahren nach einem der voranstehenden Ansprüche, **gekennzeichnet durch** das Erzeugen eines Stroms aus dem wässrigen Medium, dem das anorganische Oxidationsmittel zugesetzt ist, mit darin suspendierten ersten Partikeln, wobei die gepulste Laserstrahlung auf zumindest einen Abschnitts des Stroms gerichtet ist.

5. Verfahren nach Anspruch 4, **dadurch gekennzeichnet, dass** der Strom des Mediums mit den darin suspendierten ersten Partikeln ein freier Flüssigkeitsstrom ist.

6. Verfahren nach einem der voranstehenden Ansprüche, **dadurch gekennzeichnet, dass** die in dem wässrigen Medium suspendierten ersten Partikel bereitgestellt werden durch Erzeugung durch Aufbringen einer ersten gepulsten Laserstrahlung auf einen in einem wässrigen Medium angeordneten metallhaltigen Körper, der Gold und/oder ein Platinmetall enthält, oder durch Drahterosion eines metallhaltigen Körpers in Form eines Drahts aus Gold und/oder einem Platinmetall in dem wässrigen Medium, wobei das wässrige Medium bevorzugt frei von organischen Kohlenstoffverbindungen ist.

7. Verfahren nach einem der Ansprüche 4 bis 6, **dadurch gekennzeichnet, dass** der Durchmesser des Abschnitts des Stroms maximal so groß ist wie der auf diesen Abschnitt gerichtete Fokus der gepulsten Laserstrahlung.

8. Verfahren nach einem der voranstehenden Ansprüche, **dadurch gekennzeichnet, dass** die gepulste Laserstrahlung eine Wellenlänge von 330 bis 1500 nm bei einer Repetitionsrate von mindestens 10 Hz aufweist.

9. Verfahren nach einem der voranstehenden Ansprüche, **dadurch gekennzeichnet, dass** die gepulste Laserstrahlung eine Fluenz von zumindest 0,8 J/cm² aufweist.

10. Verfahren nach einem der voranstehenden Ansprüche, **dadurch gekennzeichnet, dass** das Medium, in dem die ersten Partikel suspendiert sind, aus Wasser mit einem darin enthaltenen anorganischen Oxidationsmittel besteht.

11. Verfahren nach einem der voranstehenden Ansprüche, **dadurch gekennzeichnet, dass** die in dem Medium suspendierten Nanopartikel, optional nach Entfernen des anorganischen Oxidationsmittels, mit einer Substanz kontaktiert werden, die ausgewählt ist aus organischen Liganden, einem anorganischen Träger und einem optisch aktiven Festkörper.

12. Verfahren nach einem der Ansprüche 6 bis 11, **dadurch gekennzeichnet, dass** der metallhaltige Körper ein reines Metall oder eine Metalllegierung der Platingruppe ist.

13. Verfahren nach einem der voranstehenden Ansprüche, **dadurch gekennzeichnet, dass** die Nanopartikel Cluster sind.

14. Metallhaltiger Nanopartikel, erhältlich durch ein Verfahren nach einem der voranstehenden Ansprüche, **dadurch gekennzeichnet, dass** die Nanopartikel in Medium, das vorzugsweise Wasser ist, in Suspension vorliegen, das frei ist von organischen Kohlenstoffverbindungen und einen Gehalt an einem anorganischen Oxidationsmittel aufweist..

15. Nanopartikel nach Anspruch 14, **dadurch gekennzeichnet, dass** sie einen mittleren Durchmesser von maximal 5 nm aufweisen.

16. Nanopartikel nach einem der Ansprüche 14 bis 15, **dadurch gekennzeichnet, dass** er an einem organischen Liganden, einem anorganischen Träger oder auf einem optisch aktiven Festkörper angeordnet ist.

17. Vorrichtung zur Herstellung von Nanopartikeln in einem Verfahren nach einem der Ansprüche 1 bis 9, mit einem Behälter (1), in dem eine Halterung zur Fixierung eines Metallkörpers (2) angeordnet ist, wobei der Behälter (1) zur Aufnahme eines Mediums (3) eingerichtet ist und ein erster Laser (4) eingerichtet ist, den in der Halterung angeordneten Metallkörper (2) mit erster Laserstrahlung (5) zu bestrahlen, **gekennzeichnet durch** eine Einrichtung zur Zuführung von Medium (3) aus dem Behälter (1) zu einer Düse (8), die zur Erzeugung eines Stroms aus dem Medium (3) mit darin enthaltenen ersten Partikeln eingerichtet ist und mit einem zweiten Laser (11), der zur Einstrahlung einer zweiten Laserstrahlung (12) auf einen Abschnitt des aus der Düse (8) austretenden Stroms (9) eingerichtet ist.

18. Vorrichtung nach Anspruch 17, **gekennzeichnet durch** eine Rückführleitung (16), die eingerichtet ist, den aus der Düse (8) austretenden Strom (9) aus Medium mit darin enthaltenen Nanopartikeln im Anschluss an den Durchtritt **durch** die zweite Laserstrahlung (12) zur Düse (8) zurückzuführen.

19. Vorrichtung nach Anspruch 17 oder 18, **gekennzeichnet durch** eine Dosiereinrichtung zur Dosierung eines anorganischen Oxidationsmittels in eine Leitung oder in einen Behälter (1), der vor der Düse (8) angeordnet ist und **durch** ein in einer Leitung vor der Düse (8) angeordnetes Spektrometer (20) oder **durch** ein nach dem Auslass (14) des Gehäuses (10) angeordnetes Spektrometer (21), wobei die Dosiereinrichtung eingerichtet ist, abhängig vom Spektrometer (20, 21) gesteuert zu werden.
